# EUROPEAN PATENT APPLICATION

(11) **EP 3 326 559 A1**
(43) Date of publication of application: **30.05.2018**
(21) Application number: 15898972.3
(22) Date of filing: 30.11.2015
(51) Int. Cl.: A61B 18/00

(54) **ULTRASOUND TREATMENT TOOL AND ULTRASOUND TREATMENT ASSEMBLY**

(30) Priority: 23.07.2015 US 201562196158 P
(71) Applicant: OLYMPUS CORPORATION, Hachioji-shi Tokyo 192-8507 (JP)
(72) Inventor: SAKAI, Masahiro, Tokyo 192-8507 (JP)
(74) Representative: Gunzelmann, Rainer
(86) International application number: PCT/JP2015/083591
(87) International publication number: WO 2017/013814

(57) **Abstract**

A ultrasonic treatment apparatus (15) includes a handpiece (41). The handpiece (41) includes a probe (44) and a hollow sheath (51). The sheath (51) covers and protects the probe (44). The probe (44) has a rod-shaped main body portion (61) and a treatment portion (61A) provided at the distal end of the main body portion (61). The treatment part (61A) includes a cutting region (63). The sheath (51) includes a first portion (64) covering the main body portion (61) and a second portion (65) covering the curved portion (62) of the treatment portion (61A). The physician inserts the probe (44) and sheath (51) into the joint space (17) and strikes the cutting area (63) against the bone (12). When ultrasonic vibration is transmitted to the probe (44), the bone (12) is cut. If the arthroscope (18) makes contact with the probe (44) during vibration, the probe (44) may be damaged. The bending part (62) is most likely to break. Therefore, the second part (65) of the sheath covers the bending part (62) and protects it from contact with the arthroscope (18).

## Description

### FIELD

The present invention relates generally to an ultrasonic treatment apparatus and an ultrasonic treatment assembly.

### BACKGROUND

International Publication No. 2010/087060 discloses a treatment system for surgery having an arthroscopic apparatus and a treatment apparatus for surgery. In the treatment system for surgery, a doctor inserts an arthroscope of the arthroscopic apparatus and a treatment tool of the treatment apparatus into a joint cavity, and performs treatment by using the treatment tool under arthroscopy in the joint cavity.

### CITATION LIST

PATENT LITERATURE 1: International Publication No. 2010/087060

### SUMMARY

### TECHNICAL PROBLEM

When an arthroscope and a treatment tool are inserted into a joint cavity which is a small space, a problem of the arthroscope and the treatment tool being brought in contact with each other may occur. For example, for treatment using an ultrasonic treatment tool which applies ultrasonic vibration, it is required to prevent a problem of the arthroscope being brought into contact with a probe to which ultrasonic vibration is transmitted.

The object of the present invention is to provide an ultrasonic treatment apparatus and an ultrasonic treatment assembly which are capable of reducing a possibility of causing a problem.

### SOLUTION TO PROBLEM

In order to accomplish the object, in an aspect of the present invention, an ultrasonic treatment apparatus used for arthroscopic surgery includes a probe which includes a main body section to which ultrasonic vibration is transmitted, and a treatment section provided in a distal end side of the main body section and having an excision region which excises a bone by the ultrasonic vibration; and a sheath which includes a first portion provided to cover the main body section of the probe, and a second portion extending from the first portion that covers a part of the treatment section closer to a proximal end side than the excision region.

### ADVANTAGEOUS EFFECTS OF INVENTION

According to the present invention, an ultrasonic treatment apparatus and an ultrasonic treatment assembly which are capable of reducing a possibility of causing a problem can be provided.

### BRIEF DESCRIPTION OF THE DRAWINGS

FIG. 1 is a schematic diagram of a treatment system according to the first embodiment of the present invention.
FIG. 2 is a side view of a handpiece of an ultrasonic treatment apparatus of the treatment system shown in FIG. 1.
FIG. 3 is a cross-sectional view of the handpiece taken along a plane in a longitudinal axis C of FIG. 2.
FIG. 4 is a cross-sectional view of an enlarged portion A of FIG. 3.
FIG. 5 is a side view illustrating a positional relationship between the ultrasonic treatment apparatus and an arthroscopic apparatus in the actual use of the treatment system shown in FIG. 1.
FIG. 6 is a side view of a handpiece of a treatment system according to the second embodiment.
FIG. 7 is a cross-sectional view of an enlarged portion B of FIG. 6.
FIG. 8 is a cross-sectional view taken along line F8-F8 in FIG. 6.
FIG. 9 is a cross-sectional view of a handpiece taken along a plane in a longitudinal axis C of the treatment system according to a third embodiment.
FIG. 10 illustrates the handpiece viewed from an arrow E shown in FIG. 9.

### DETAILED DESCRIPTION

### [First Embodiment]

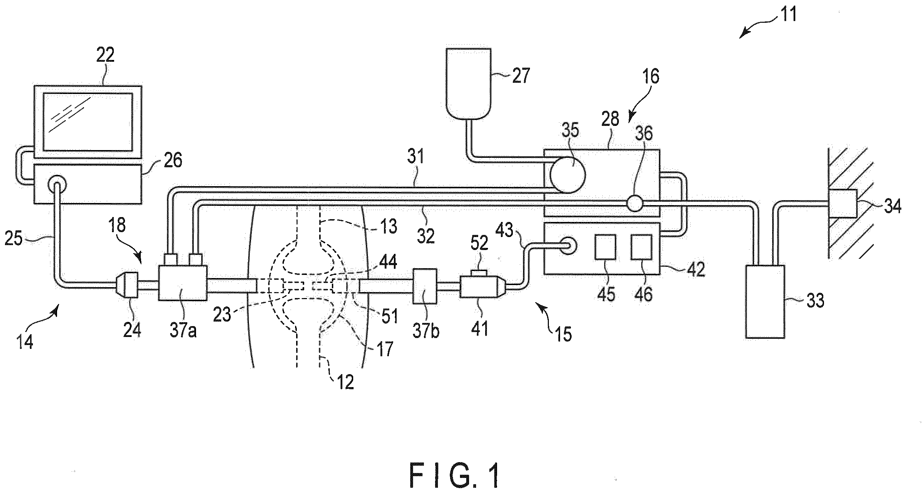

The first embodiment of the present invention will be explained with reference to FIG. 1 to FIG. 5. The treatment system is used for arthroscopic surgery performed for a target region such as a joint of a shoulder, knee, elbow, etc. Specifically, as shown in FIG. 1, a treatment system 11 is used for treatment within a joint, namely, between a first bone 12 and a second bone 13. The treatment system 11 includes an arthroscopic apparatus 14, an ultrasonic treatment apparatus 15 (ultrasonic treatment assembly), and a perfusion apparatus 16. An arthroscope 18 of the arthroscopic apparatus 14 is inserted into the joint cavity 17 through a first cannula 37a, and a sheath 51 and a probe 44 (described later) of the ultrasonic treatment apparatus 15 are inserted into a joint cavity 17 through a second cannula 37b.

The arthroscopic apparatus 14 is provided with the arthroscope 18 which monitors an inside of a joint of a patient, namely, the joint cavity 17, an image processing unit 26 that performs image processing based on a subject image captured by the arthroscope 18, and a monitor 22 that displays an image generated by the image processing at the image processing unit 26.

The arthroscope 18 includes an insertion section 23 and a holding section 24. In a treatment using the arthroscope 18, a distal end portion of the insertion section 23 is inserted into a joint. One end of a universal cord 25 is connected to the holding section 24. The other end of the universal cord 25 is connected to the image processing unit 26. The image processing unit 26 is electrically coupled to a monitor 22 (display unit).

An imaging element is provided at a distal end portion of the insertion section 23. The imaging element is electrically connected to the image processing unit 26. An image captured by the imaging element is subjected to image processing at the image processing unit 26, and is displayed on the monitor 22. The arthroscope 18 is coupled to a light source unit (not shown in the drawings), and light emitted from the light source unit is applied to a subject.

The perfusion apparatus 16 includes an inflatable liquid source 27 in which perfusate such as a saline solution is stored, a perfusion pump unit 28, a liquid transfer tube 31, one end of which is connected to the liquid source 27, a drainage tube 32, and a suction bottle 33 connected to one end of the drainage tube 32. The suction bottle 33 is connected to a suction pump unit 34 attached to a wall of an operation room. The perfusion pump unit 28 is capable of transferring perfusate from the liquid source 27 by means of a liquid transfer pump 35. The perfusion pump unit 28 is capable of switching suction and stopping suction of perfusate within the joint cavity 17 relative to the suction bottle 33 by opening or closing a pinch valve 36 used as a drainage valve.

The liquid transfer tube 31, which is a liquid transfer path, is connected to the first cannula 37a at the other end. The liquid transfer tube 32 is connected to the first cannula 37a at the other end. Accordingly, it is possible to transfer perfusate into the joint cavity 17 or drain perfusate from the joint cavity 17 through the first cannula 37a. It may be possible to provide a portal to a patient with which perfusate can be transferred or drained.

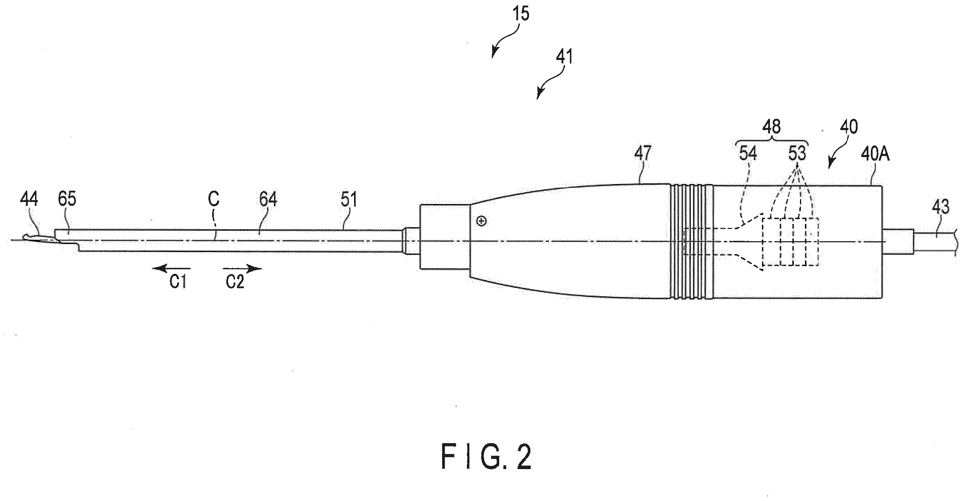

As shown in FIGS. 1 and 2, the ultrasonic treatment apparatus 15 is provided with a handpiece (ultrasonic treatment apparatus) 41, an ultrasonic vibrator unit 40 attached to the handpiece 41, a power supply unit 42, a cable 43 which connects the ultrasonic vibrator unit 40 and the power supply unit 42. In the following description, it is assumed that one of the directions in a longitudinal axis C of the probe 44 (main body section 61) provided to the handpiece 41 is a distal end direction C1, and another direction which is opposite to the distal end direction C1 is a proximal end direction C2, as shown in FIG. 2.

As shown in FIG. 1, the power supply unit 42 is provided with an energy controller 45, and an ultrasonic current supply unit 46 which is controlled by the energy controller 45 to supply power to a vibration generator. The ultrasonic vibrator unit 40 has a vibration generator 48 which generates ultrasonic vibration, inside of a vibrator case 40A.

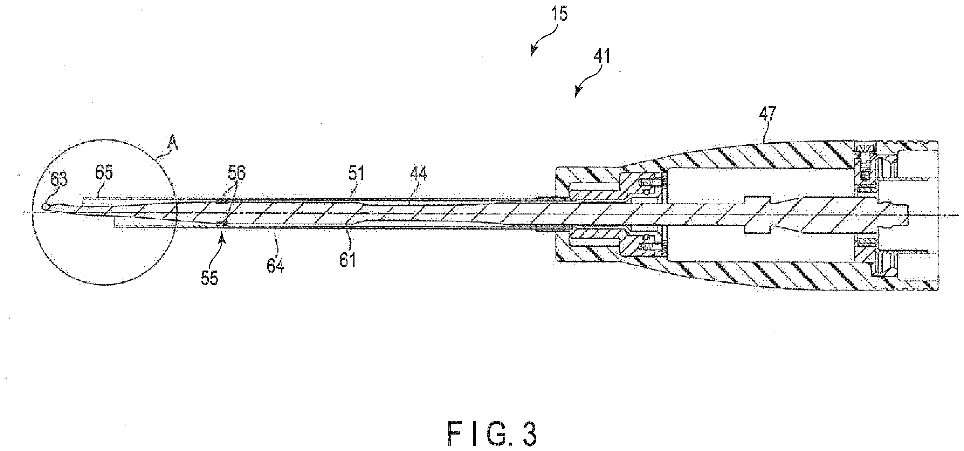

As shown in FIGS. 1 to 3, the handpiece (ultrasonic treatment apparatus) 41 is provided with a housing 47 constituting an outer shell, the rod-like probe 44 connected to the vibration generator 48, the hollow (cylindrical) sheath 51 which covers the periphery of the probe 44 to protect the probe 44, and an energy input button 52 (switch) provided to the housing 47. The energy input button 52 is not shown in FIGS. 2 and 3. The energy input button 52 may be provided as a foot switch separated from the handpiece 41.

The vibrator case 40A (piezoelectric element 53) is connected to one end of a cable 43. The other end of the cable 43 is connected to the power supply unit 42. If a doctor operates the energy input button 52, the energy controller 45 senses an input operation of the energy input button 52. The energy controller 45 then controls the ultrasonic current supply unit 46 to supply power to the vibration generator 48. By this operation, ultrasonic vibration (ultrasonic energy) is transmitted to the probe 44, and the ultrasonic vibration is transmitted through the probe 44 to a bone (living tissue) which is a treatment target. Accordingly, excision or removal treatment, for example, can be applied to a bone (living tissue).

The vibration generator 48 is provided with a plurality of piezoelectric elements (vibrators) 53 and a horn member 54. The piezoelectric elements 53 generate ultrasonic vibration upon receipt of power supplied from the power supply unit 42. The horn member 54 increases an amplitude of the ultrasonic vibration generated at the piezoelectric elements 53 and transmits the ultrasonic vibration to the probe 44. The ultrasonic vibration in the direction along the longitudinal axis C (the direction in which the probe 44 is shortened and extended) is, for example, transmitted to the horn member 54 and the probe 44 connected to the horn member 54. A resin sealing member 56 that prevents liquid from entering the inside of the sheath 51 is provided at a position corresponding to a node position 55 (a node position closest to the distal end direction C1 side) of the ultrasonic vibration traveling on the probe 44 as stated above. The sealing member 56 has a ring-like shape, and supports the probe 44 so that the probe 44 is positioned at the center of the sheath 51.

As shown in FIGS. 2 to 4, the probe 44 is formed of a metallic material having biocompatibility (for example, a titanium alloy, etc.) as a rod-like shape. The probe 44 has a main body section 61 extending as a rod-like shape, and a treatment section 61A (distal end direction C1 side section) provided at the distal end direction C1 side of the main body section 61. The treatment section 61A is bent in a direction different from the direction along the longitudinal axis C. The treatment section 61A has a bending portion 62 provided in a part close to the distal end direction C1 of the main body section 61, and an excision region 63 (claw portion) extending in a direction crossing the longitudinal axis C from the part closer to the distal end direction C1 side than the bending portion 62, and excises a bone by transmission of ultrasonic vibration. The treatment section 61A has a diameter smaller than the other portions of the probe 44, and is easier to be broken then the other portions. In particular, for removal of a hard tissue such as bone, an amplitude of ultrasonic vibration is set to be relatively large, and a stress applied to the probe 44 is relatively large. The treatment section 61A receives a relatively large stress in the same manner.

The main body section 61 is capable of transmitting ultrasonic vibration received from the vibration generator 48 from the proximal end to the distal end direction C1 side. The treatment section 61A is bent to protrude in the direction crossing the longitudinal axis C of the main body section 61. The bending portion 62 (top portion) has a largest curvature in the main body section 61 of the probe 44. While ultrasonic vibration is applied during treatment, the bending portion 62 is easily broken in a case of coming into contact with the arthroscope 18.

The wavelength of ultrasonic vibration to be input to the probe 44 is determined based on a resonant frequency of the piezoelectric elements 53 of the ultrasonic vibrator unit 40. That is, the length of the probe 44 is determined based on the piezoelectric elements 53 to be used. Thus, the probe 44 has a length so that an anti-node position of vibration is determined at the treatment section 61A when ultrasonic vibration is input to the proximal end of the probe 44 from the piezoelectric elements 53 of the ultrasonic vibrator unit 40, and the ultrasonic vibration is transmitted to the distal end direction C1 side from the proximal end of the probe 44. The anti-node position of vibration preferably matches the position of the excision region 63. That is, the position of the excision region 63 is adjusted to correspond to the anti-node position of vibration. When ultrasonic vibration is transmitted, a first node position of vibration at the proximal end side relative to the anti-node position of vibration is defined between the proximal end and the distal end of the main body section 61 of the probe 44.

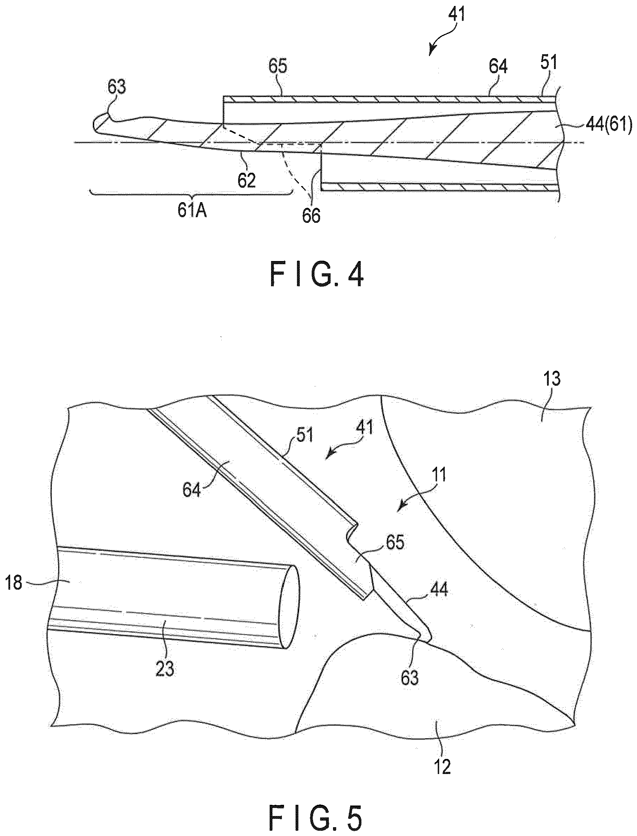

The sheath 51 includes a first portion 64 having a tubular shape such as a cylindrical shape, and a second portion 65 extending from the first portion 64. The second portion 65 is provided at a side where the excision region 63 is provided. As shown in FIG. 4, the second portion 65 is provided to project from the first portion 64 and to have substantially half of the size of the cylinder corresponding to 180°around the longitudinal axis C, for example. The second portion 65 extends from the first portion 64 to the position to cover a portion corresponding to the bending portion 62 of the probe 44. The distal end of the second portion 65 extends in the distal end direction C1 over the bending portion 62 in comparison with the distal end of the first portion 64. On the other hand, the second portion 65 is not provided to the side opposite at the side where the excision region 63 is defined, and the probe 44 is externally exposed at this position. Accordingly, the side opposite to the side corresponding to the second portion 65 of the proximal end portion of the treatment section 61A of the probe 44 is defined as an exposed portion 66.

Next, a surgical method using the treatment system 11 according to the present embodiment will be described with reference to FIG. 5. The ultrasonic treatment apparatus 15 of the treatment system 11 according to the present embodiment is used within the joint cavity 17, for example, and is capable of resecting and removing a tissue to be removed (removal target) by bringing the probe 44 to which ultrasonic vibration is transmitted into contact with a removal target such as a bone spur.

First, the joint cavity 17 is filled with perfusate by a well-known method. A suitable portal that reaches into the joint cavity 17 from the outside is used to insert the arthroscope 18 into the joint cavity 17 to check the state of a patient. A doctor uses another portal to insert the probe 44 and the sheath 51 of the handpiece 41 into the joint cavity 17. A plurality of well-known portals which are access paths to the inside of a joint are present in each joint.

When inserting the probe 44 into the joint cavity 17, the probe 44 to which ultrasonic vibration is transmitted can be used for removal of intervening soft tissue such as a synovial membrane, synovial bursa, cartilage, etc. Accordingly, when inserting the probe 44 and the sheath 51 into the joint cavity 17, there is no need to switch treatment tools, thereby improving workability.

As shown in FIG. 5, there is a case where a doctor places the probe 44 and the arthroscope 18 so that the distal end portion of the insertion section 23 of the arthroscope 18 is placed close to the excision region 63 side of the probe 44. The doctor can transmit ultrasonic vibration to the probe 44 when pressing the energy input button 52 while the excision region 63 of the probe 44 is brought into contact with a bone spur, etc. of the bone 12 (living tissue) to be treated under the operation of the arthroscope 18. By this process, removal or excision of a hard tissue to be treated such as a bone can be accomplished. At this time, it is likely that the insertion section 23 of the arthroscope 18 is placed close to the probe 44 as shown in FIG. 5. In such a case, the excision region 63 side of the probe 44 is protected by the second portion 65 extending relative to the first portion 64 of the sheath 51. Accordingly, the ultrasonically vibrating probe 44 is prevented from coming into contact with the insertion section 23 of the arthroscope 18. Thus, in the case where the insertion section 23 of the arthroscope 18 is located near the excision region 63 of the probe 44, the insertion section 23 of the arthroscope 18 is placed close to or in contact with the sheath 51, to be prevented from coming in contact with the probe 44, thereby preventing the probe 44 from being broken (or damaging the arthroscope 18) at the treatment section 61A, for example.

According to the first embodiment, the ultrasonic treatment apparatus 15 used for surgery using the arthroscope 18 comprises; the probe 44 including the main body section 61 to which ultrasonic vibration is transmitted, the excision region 63 which is provided at the distal end side of the main body section 61 and excises a bone by the ultrasonic vibration, the sheath 51 including the first portion 64 that covers the main body section 61 of the probe 44, and the second portion 65, which extends from the first portion 64 to cover the side where the excision region 63 of the main body section 61 is provided.

With this configuration, the second portion 65 is provided to cover the side where the excision region 63 of the probe 44 is provided, thereby preventing a problem that the probe 44 to which ultrasonic vibration is transmitted is in contact with the arthroscope 18.

The main body section 61 has the bending portion 62 that is bent relative to the longitudinal axis C of the main body section 61, and the second portion 65 of the sheath 51 covers the side of the bending portion 62 where the excision region 63 is defined. With this configuration, the portion corresponding to the bending portion 62 of the probe 44 which has the largest curvature and is easiest to be broken can be protected by the second portion 65. Accordingly, even if a doctor unintentionally brings the probe 44 close to the arthroscope 18 during surgery, the arthroscope 18 is not brought into contact with the portion corresponding to the bending portion 62 of the probe 44. Thus, the probe 44 can be prevented from being broken at a position where the bending portion 62 is provided. In addition, the second portion 65 has a shape of substantially half of the cylinder which can be simply accomplished. Therefore, even if the second portion 65 is provided, an increase in the manufacturing costs can be suppressed.

### [Second Embodiment]

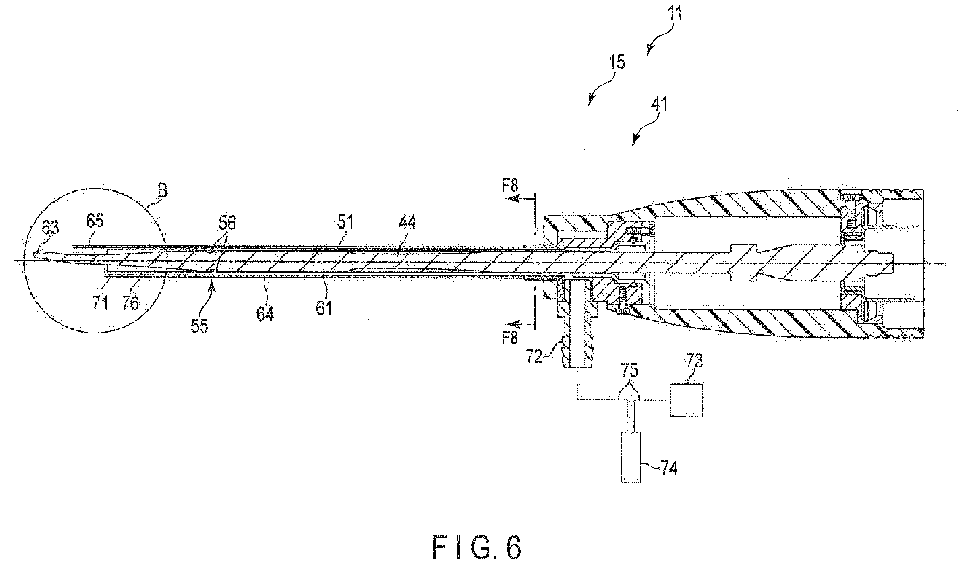

A treatment system 11 according to the second embodiment will be described with reference to FIGS. 6 to 8. The treatment system 11 of the second embodiment is different from the treatment system 11 of the first embodiment in that a second sheath 76 and a suction path 71 are provided inside of a sheath 51 of an ultrasonic treatment apparatus 15, but the other structures are identical to those of the first embodiment. Accordingly, mainly portions different from the first embodiment will be explained, and portions the same as the first embodiment will not be explained or shown in the drawings.

As shown in FIGS. 6 to 8, a handpiece 41 of the ultrasonic treatment apparatus 15 includes a rod-like probe 44, a hollow (a tubular shape such as a cylindrical shape) sheath 51 which covers the periphery of the probe 44 to protect the probe 44, a second sheath 76 (tubular member) which is a tubular shape such as a cylindrical shape and provided inside of the sheath 51, a suction path 71 provided between the sheath 51 and the second sheath 76, a connection cap 72 connected to the suction path 71, and an energy input button 52 (switch) provided to a housing 47.

As shown in FIG. 6, the ultrasonic treatment apparatus 15 is also provided with a suction source 73 which includes a vacuum pump, etc, and causes negative pressure in the suction path, and with a tank 74 to store suctioned liquid or living tissue between the suction path 71 and the suction source 73. The suction path 71 is connected to the suction source 73 through the connection cap 72 and a tube 75 connected thereto.

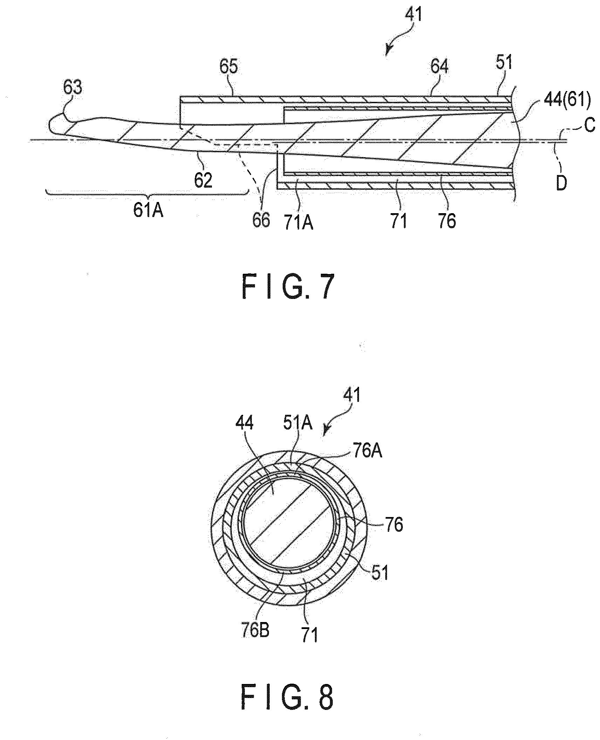

As shown in FIGS. 6 and 7, the second sheath 76 (tubular member), which is an inner sheath, is interposed between the first portion 64 of the sheath 51, which is an outer sheath, and the probe 44. The second sheath 76 is provided to cover the probe 44 (main body section 61). In the embodiment, the second sheath 76 is arranged to be eccentric relative to a central axis D of the sheath 51. Thus, a central axis C of the second sheath 76 is shifted relative to the central axis D of the sheath 51. As shown in FIG. 8, the second sheath 76 includes a proximal part 76A close to a part 51A on an inner peripheral surface of the sheath 51, and a distal part 76B opposite to the proximal part 76A.

As shown in FIGS. 7 and 8, the suction path 71 is provided in a space between the inner side of the first portion 64 of the sheath 51 and the outer side of the second sheath 76. Specifically, the suction path 71 is provided between the distal part 76B and the inner peripheral surface of the sheath 51 (positioned away from the part 51A of the inner peripheral surface of the sheath 51). The suction path 71 includes a suction opening 71A exposed to the outside at the distal end direction C1 side.

A surgical method using the treatment system 11 according to the present embodiment will be described with reference to FIGS. 6 to 8.

A joint cavity 17 is filled with perfusate by a well-known method. An arthroscope 18 is inserted into the joint cavity 17 from a suitable portal to check the condition of a patient. A doctor uses another portal to insert the probe 44 and the sheath 51 of the handpiece 41 into the joint cavity 17. When inserting the probe 44 into the joint cavity 17, the probe 44 to which ultrasonic vibration is transmitted can be used for removal of an intervening soft tissue such as a synovial membrane, synovial bursa, cartilage, etc., similar to the first embodiment.

As shown in FIG. 5, a doctor places the probe 44 and the arthroscope 18 so that the distal end portion of the arthroscope 18 is placed near the sheath 51 at the excision region 63 side of the probe 44. The doctor can transmit ultrasonic vibration to the probe 44 when pressing the energy input button 52 while the excision region 63 of the probe 44 is brought into contact with a bone spur, etc. of the bone 12 (living tissue) to be treated under the operation of the arthroscope 18. By this process, removal or excision of a hard tissue to be treated, such as a bone can be accomplished. In this case, the excision region 63 side of the probe 44 (portion corresponding to a bending portion 62) is protected by a second portion 65 of the sheath 51. Accordingly, the probe 44 is prevented from being broken by coming in contact with the arthroscope 18 while ultrasonic vibration is transmitted to the probe 44.

According to the present embodiment, in the state where the ultrasonic treatment apparatus 15 of the treatment system 11 is powered on, the suction source 73 is in continuous operation, and a fragment of a living tissue or an air bubble generated at the excision region 63 can be suctioned and removed through the suction source 73. In addition, according to the present embodiment, since the second sheath 76 is provided to be eccentric relative to the central axis D of the sheath 51, the diameter of the suction path 71 and the suction opening 71A is ensured to be relatively large. Thus, it is possible to suction a fragment of a bone which is relatively large through the suction opening 71A and the suction path 71.

According to the present embodiment, a tubular member provided to cover the probe 44 between the first portion 64 of the sheath 51 and the probe 44, and the suction path 71 provided in a space between the first portion 64 and the tubular member and connected to the suction source 73 are provided.

With this configuration, a fragment of a living tissue or an air bubble generated by treatment can be removed through the suction path 71, thereby ensuring a clear view under an arthroscope. In particular, a fragment can be suctioned and removed near a place where the fragment is generated (the excision region 63 of the probe 44), thereby removing the fragment before being diffused to the periphery. Accordingly, it is possible to remove a fragment efficiently and to reliably provide a clear view under an arthroscope while preventing cloudiness of the perfusate. Small fragments such as powdered fragments rise to obstruct a field of view of the arthroscope 18. Accordingly, it is extremely efficient to remove such fragments near the excision region 63 in order to perform surgery effectively and safely.

The tubular member includes the proximal part 76A provided to be eccentric relative to the central axis D of the sheath 51 and close to the part 51A on an inner peripheral surface of the sheath 51, and the distal part 76B opposite to the proximal part 76A, and the suction path 71 is provided between the distal part 76B and a position away from the part 51A on an inner peripheral surface of the sheath 51.

With this configuration, the diameter of the suction path 71 is ensured to be large in comparison with the case where the tubular member is provided so that the central axis C of the tubular member matches the central axis D of the sheath 51. Accordingly, it is possible to suction and remove not only small powdered fragments, but also a fragment having a relatively large diameter, thereby improving fragment removal efficiency. Therefore, it is possible to ensure a clear view under the arthroscope 18 and to promote safety of surgery.

### [Third Embodiment]

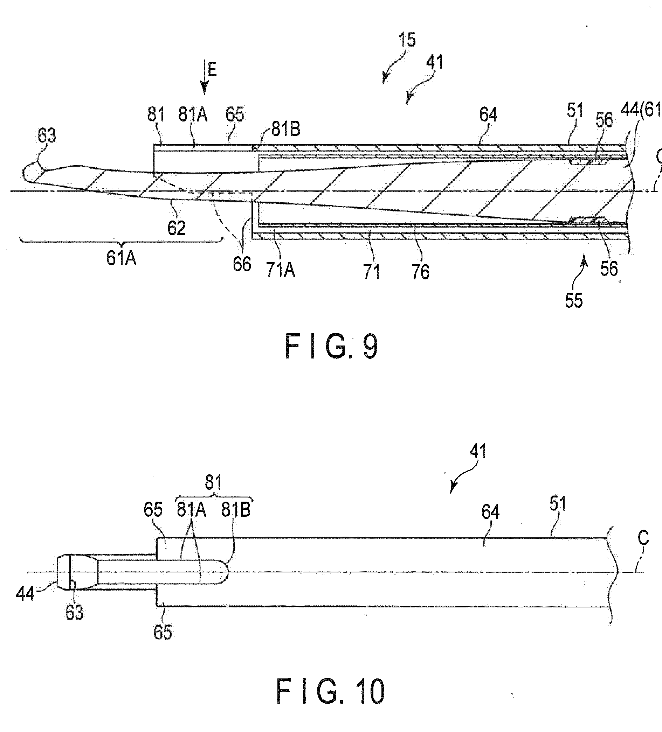

A treatment system according to the third embodiment will be described with reference to FIGS. 9 and 10. The treatment system 11 of the third embodiment is different from the treatment system 11 of the second embodiment in that a second portion 65 of a sheath 51 of an ultrasonic treatment apparatus 15 has a notch 81, but the other structures are identical to those of the second embodiment. Accordingly, mainly portions different from the second embodiment will be explained, and portions the same as the second embodiment will not be explained or shown in the drawings.

As shown in FIGS. 9 and 10, the sheath 51 includes a first portion 64 having a tubular shape such as a cylindrical shape, and a second portion 65 extending from the first portion 64. The second portion 65 is provided at a side where an excision region 63 is provided. For example, the second portion 65 is provided to correspond to an angle of 180°around the longitudinal axis C. The second portion 65 has a notch 81 which is a slit-like portion extending toward the proximal end direction C2 from the distal end of the second portion 65. The notch 81 includes a main body part 81A extending linearly along the longitudinal axis C, and an arch-shaped bottom part 81B provided at an end of the proximal end direction C2 side of the main body part 81A. The length of the notch 81 along the longitudinal axis C is substantially the same as the length of the second portion 65 along the longitudinal axis C.

The notch 81 may be provided to span across the second portion 65 and the first portion 64, not only in the second portion 65. In this case, it is preferable that the notch 81 extends from the distal end portion of the second portion 65 to the proximal end direction C2 side, and the bottom part 81B of the notch 81 is located closer to the distal end direction C1 side than a node position 55 (a node position closest to the distal end direction C1 side) of ultrasonic vibration transmitted to the probe 44.

The width in the direction crossing the longitudinal axis C of the notch 81 is set to be smaller than the diameter of an arthroscope 18 to be used. More specifically, a diameter D1 of the arthroscope 18 is set to be within the range of 2<D1≤10(mm), for example, while a size D2 of the notch 81 is set to be within the range of 1≤D2≤2(mm), for example.

A surgical method using the treatment system 11 according to the present embodiment will be described with reference to FIGS. 9 and 10.

A joint cavity 17 is filled with perfusate by a well-known method. The arthroscope 18 is inserted into the joint cavity 17 from a suitable portal to check the condition of a patient. A doctor uses another portal to insert the probe 44 and the sheath 51 of the handpiece 41 into the joint cavity 17. When inserting the probe 44 into the joint cavity 17, the probe 44 to which ultrasonic vibration is transmitted can be used for removal of intervening soft tissue such as a synovial membrane, synovial bursa, cartilage, etc., similar to the other embodiments.

As shown in FIG. 5, a doctor places the probe 44 and the arthroscope 18 so that the distal end portion of the arthroscope 18 is placed near the sheath 51 at the excision region 63 side of the probe 44. The doctor can transmit ultrasonic vibration to the probe 44 when pressing an energy input button 52 while the excision region 63 of the probe 44 is brought into contact with a bone spur, etc. of the bone 12 (living tissue) to be treated under the operation of the arthroscope 18. By this process, removal or excision of a hard tissue to be treated such as a bone can be accomplished. In this case, the excision region 63 side of the probe 44 is protected by the second portion 65 of the sheath 51. Accordingly, the probe 44 is prevented from being broken by coming in contact with the arthroscope 18 while ultrasonic vibration is transmitted to the probe 44.

In the present embodiment, the slit-like notch 81 is provided to the second portion 65. Accordingly, even if an operation is performed under the condition shown in FIG. 5, the probe 44 and the excision region 63 can be visually recognized through the notch 81 under the arthroscope 18. Accordingly, the present embodiment realizes further improvement of efficiency and safety in the surgery.

Furthermore, according to the present embodiment, in the state where the ultrasonic treatment apparatus 15 of the treatment system 11 is powered on, a suction source 73 is perpetually actuated, and a fragment of a living tissue or an air bubble generated at the excision region 63 can be suctioned and removed through the suction source 73. In addition, according to the present embodiment, since a second sheath 76 is provided to be eccentric relative to the central axis D of the sheath 51, the diameter of a suction path 71 and a suction opening 71A is ensured to be large so that a large fragment can be suctioned, similar to the second embodiment.

According to the present embodiment, the second portion 65 has the notch 81 which extends toward the proximal end direction C2 side which is opposite to the distal end direction C1 side of a main body section 61. With this configuration, when the arthroscope 18 is used at the excision region 63 side of the probe 44 for operation, the probe 44 and the excision region 63 are not hidden by the second portion 65 for protecting the probe 44, and the visual recognition of the probe 44 and the excision region 63 can be improved. Accordingly, a doctor can perform surgery efficiently and safely.

Furthermore, the width of the notch 81 in the direction crossing the longitudinal axis C of the notch 61 is set to be smaller than the diameter of the arthroscope 18 to be used in the arthroscopic surgery. With this configuration, the arthroscope 18 is not erroneously inserted into the notch 81 even if the notch 81 is provided to the second portion 65. Thus, a problem of the arthroscope 18 and the probe 44 coming in contact with each other due to the notch 81 can be prevented.

The present invention is not limited to the above-described embodiments, and can be modified in various manners when reduced to practice, without departing from the gist of the invention. In addition, the treatment system 11 according to each embodiment can of course be combined to function as a treatment system.

### REFERENCE SIGNS LIST

11: Treatment system; 14: Arthroscopic apparatus; 15: Ultrasonic treatment apparatus; 18: Arthroscope; 44: Probe; 51: Sheath; 61: Main body section; 62: Bending portion; 63: Excision region; 64: First portion; 65: Second portion; 71: Suction path; 76: Second sheath; 76A: Proximal part; 78B: Distal part; 81: Notch

## Claims

1. An ultrasonic treatment apparatus for use in arthroscopic surgery, comprising:
a probe including a main body section to which ultrasonic vibration is transmitted, and a treatment section provided in a distal end side of the main body section and including an excision region that excises a bone by the ultrasonic vibration; and
a sheath including a first portion provided to cover the main body section of the probe, and a second portion extending from the first portion to cover a part of the treatment section closer to a proximal end side than the excision region.

2. The ultrasonic treatment apparatus according to claim 1, wherein:
the treatment section includes a bending portion that is bent relative to a longitudinal direction of the main body section, and
the second portion of the sheath covers a side of the bending portion where the excision region is provided.

3. The ultrasonic treatment apparatus according to claim 1, wherein a notch extending toward the proximal end side which is opposite to the distal end side of the main body section is provided to the second portion.

4. The ultrasonic treatment apparatus according to claim 3, wherein a width of the notch in a direction crossing a longitudinal direction of the main body section is set to be smaller than a diameter of an arthroscope to be used in the arthroscopic surgery.

5. The ultrasonic treatment apparatus according to claim 1, comprising:
a tubular member provided to cover the main body section of the probe between the first portion of the sheath and the probe; and
a suction path provided between the first portion of the sheath and the tubular member and connected to a suction source.

6. The ultrasonic treatment apparatus according to claim 5, wherein:
the tubular member includes a proximal part provided to be eccentric to a central axis of the sheath and close to a part on an inner peripheral surface of the sheath, and a distal portion provided at a side opposite to the proximal part, and
the suction path is provided between the distal part and a position away from the part of the inner peripheral surface of the sheath.

7. An ultrasonic treatment assembly comprising:
the ultrasonic treatment apparatus according to claim 1; and
an ultrasonic vibrator unit detachably attached to the ultrasonic treatment apparatus.
